# EUROPEAN PATENT APPLICATION

(11) **EP 4 537 850 A1**
(43) Date of publication of application: **16.04.2025**
(21) Application number: 23820164.4
(22) Date of filing: 12.06.2023
(51) Int. Cl.: A61K 47/42, A61K 47/18, A61K 47/22, A61K 38/48, C07K 14/00

(54) **COMPOSITION FOR STABILIZING BOTULINUM NEUROTOXIN, BOTULINUM NEUROTOXIN FORMULATION CONTAINING SAME, AND POLYPEPTIDES FOR USE THEREIN**

(30) Priority: 10.06.2022 KR 20220070554
(71) Applicant: Medytox Inc., Chungcheongbuk-do 28126 (KR)
(72) Inventor: KIM, Suhyeok, Suwon-si, Gyeonggi-do 16529 (KR); LEE, Dongkyu, Suwon-si, Gyeonggi-do 16493 (KR); KWAK, Seongsung, Suwon-si, Gyeonggi-do 16228 (KR)
(74) Representative: Brevalex
(86) International application number: PCT/KR2023/008066
(87) International publication number: WO 2023/239229

(57) **Abstract**

Provided are a composition for stabilizing botulinum neurotoxin, a botulinum neurotoxin formulation including the same, and a polypeptide for use therein.

## Description

### Technical Field

The present disclosure relates to a composition for stabilizing a botulinum neurotoxin, a botulinum neurotoxin formulation including the same, and a polypeptide for use therein.

### Background Art

Botulinum neurotoxin (BoNT), as a polypeptide product of *Clostridium botulinum* that is an anaerobic bacterium, is a toxic substance specifically acting on nerve cells. Although BoNT is originally a lethal toxic substance, it has been used for treatment of cervical dystonia (CD), blepharospasm, hyperhidrosis, strabismus, achalasia, neurogenic bladder, urologic disease, migraine, and the like.

International Publication No. WO2008/082889 discloses a method and composition for stabilizing a botulinum toxin by using a polypeptide that is a HIV-TAT fragment or a derivative of a HIV-TAT fragment.

International Publication No. WO2010/078242 discloses an injectable composition in which a positively charged carrier having a specific amino acid sequence is linked to botulinum toxin via non-covalent bonds.

An alternative method of stabilizing BoNT by inhibiting diffusion thereof is required.

### Disclosure of Invention

### Technical Problem

Provided is a composition for stabilizing a botulinum neurotoxin including a polypeptide inhibiting diffusion of the botulinum neurotoxin *in vivo.*

Provided is a botulinum neurotoxin formulation including a botulinum neurotoxin and the composition for stabilizing the botulinum neurotoxin.

Provided is a polypeptide for use in the composition or formulation.

### Solution to Problem

According to an aspect, a composition for stabilizing a botulinum neurotoxin includes a polypeptide inhibiting diffusion of the botulinum neurotoxin *in vivo* and represented by Formula 1 below.

[Formula 1] (C)ₙ-V

In the formula, C represents a moiety interacting with a cell membrane of a nerve cell, V represents a moiety interacting with a cell membrane of a nerve cell and interacting with the botulinum neurotoxin, and n is an integer of 0 or 1.

According to another aspect, a botulinum neurotoxin formulation includes a botulinum neurotoxin and the composition for stabilizing the botulinum neurotoxin.

According to another aspect, a polypeptide consists of the amino acid sequence of SEQ ID NO: 15, SEQ ID NO: 16, SEQ ID NO: 17, SEQ ID NO: 18, SEQ ID NO: 19, or SEQ ID NO: 20.

### Advantageous Effects of Invention

According to the present disclosure, a composition for stabilizing a botulinum neurotoxin, which improves stability by inhibiting diffusion of the botulinum neurotoxin *in vivo,* a botulinum neurotoxin formulation including the same, and a polypeptide for use therein are provided.

A botulinum neurotoxin formulation according to the present disclosure may represent one or more advantages of reduced unwanted diffusion after injection, increased duration of clinical efficacy, improved safety, or reduced antigenicity, compared to common botulinum neurotoxin formulations.

### Brief Description of Drawings

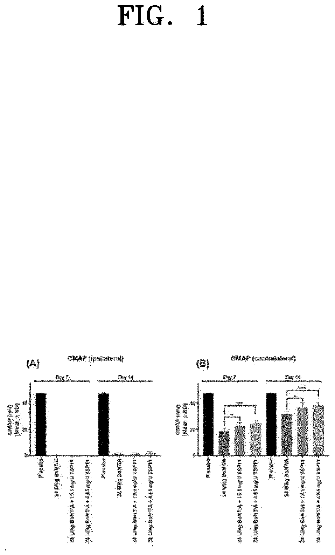

FIG. 1 is a graph showing CMAP values of a muscle of the administered site (A) and a contralateral muscle of the non-administered site (B) on day 7 and day 14 after administering placebo, botulinum neurotoxin type A (BoNT/A, Coretox inj.), and botulinum neurotoxin type A + TSP11 (BoNT/A + TSP11) to the right gastrocnemius muscle of the mice described in Example 1. CMAP values were analyzed by using a two-tailed t-test (*p<0.05 and ***p<0.001).

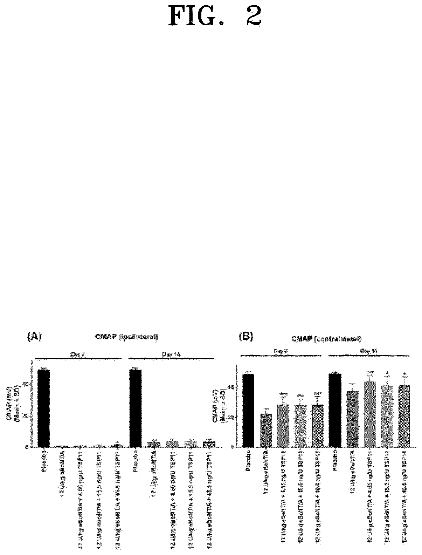

FIG. 2 is a graph showing CMAP values of a muscle of the administered site (A) and a contralateral muscle of the non-administered site (B) on day 7 and day 14, after administering placebo, recombinant botulinum neurotoxin type A (eBoNT/A), and recombinant botulinum neurotoxin type A + TSP11 (eBoNT/A + TSP11) to the right gastrocnemius muscle of the mice described in Example 2. CMAP values were analyzed by using a two-tailed t-test (*p<0.05 and ***p<0.001 vs. eBoNT/A-administered group).

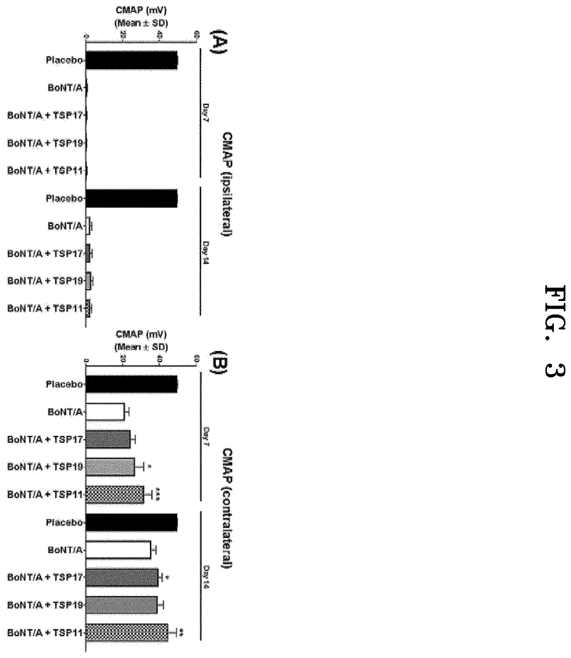

FIG. 3 is a graph showing CMAP values of a muscle of the administered site (A) and a muscle of the non-administered site (B) on day 7 and day 14, after administering placebo, botulinum neurotoxin type A (BoNT/A, Coretox inj.), botulinum neurotoxin type A + TSP17 (BoNT/A + TSP17), botulinum neurotoxin type A + TSP19 (BoNT/A + TSP19), and botulinum neurotoxin type A + TSP11 (BoNT/A + TSP11) to the right gastrocnemius muscle of the mice described in Example 5. CMAP values were analyzed by using a two-tailed t-test (*p<0.05, **p<0.01, and ***p<0.001).

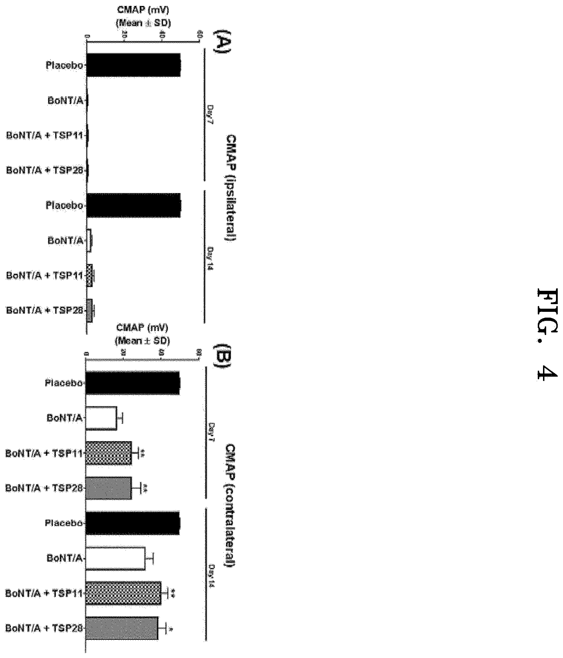

FIG. 4 is a graph showing CMAP values of a muscle of the administered site (A) and a contralateral muscle of the non-administered site (B) on day 7 and day 14, after administering placebo, botulinum neurotoxin type A (BoNT/A, Coretox inj.), botulinum neurotoxin type A + TSP11 (BoNT/A + TSP11), and botulinum neurotoxin type A + TSP28 (BoNT/A + TSP28) to the right gastrocnemius muscle of the mice described in Example 6. CMAP values were analyzed by using a two-tailed t-test (*p<0.05 and **p<0.01).

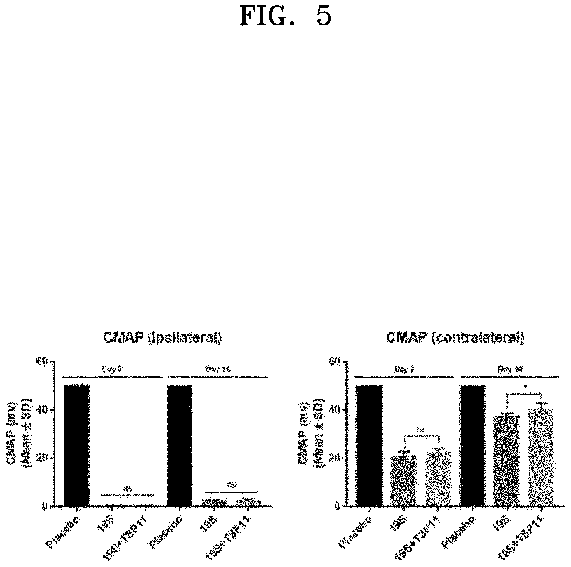

FIG. 5 is a graph showing CMAP values of a muscle of the administered site (A) and a contralateral muscle of the non-administered site (B) on day 7 and day 14, after administering placebo, botulinum neurotoxin type A (BoNT/A, 19S), and botulinum neurotoxin type A + TSP11 (BoNT/A, 19S + TSP11) to the right gastrocnemius muscle of the mice described in Example 7. CMAP values were analyzed by using a two-tailed t-test (*p<0.05 and ns; not significant).

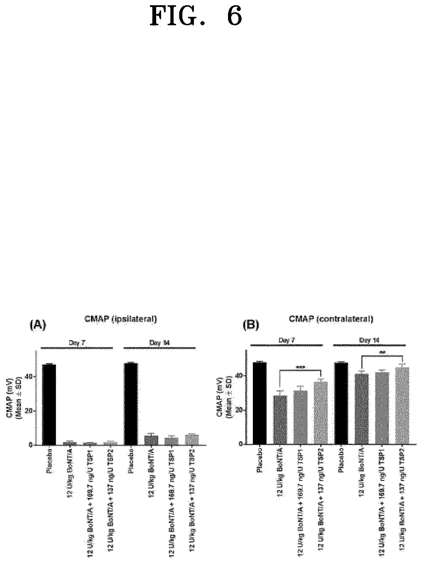

FIG. 6 is a graph showing CMAP values of a muscle of the administered site (A) and a contralateral muscle of the non-administered site (B) on day 7 and day 14, after administering placebo, botulinum neurotoxin type A (BoNT/A, Coretox inj.), botulinum neurotoxin type A + TSP1 (BoNT/A + TSP1), and botulinum neurotoxin type A + TSP2 (BoNT/A + TSP2) to the right gastrocnemius muscle of the mice described in Example 8. CMAP values were analyzed by using a two-tailed t-test (**p<0.01 and ***p<0.001).

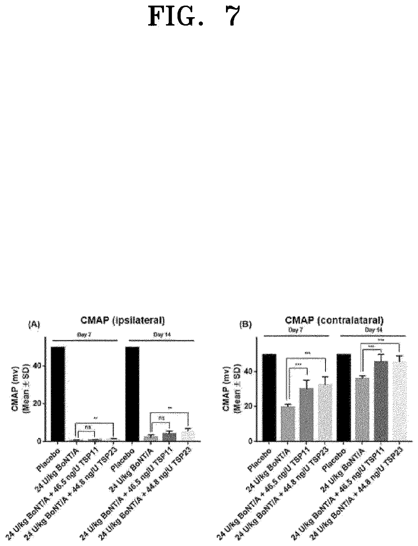

FIG. 7 is a graph showing CMAP values of a muscle of the administered site (A) and a contralateral muscle of the non-administered site (B) on day 7 and day 14, after administering placebo, botulinum neurotoxin type A (BoNT/A, Coretox inj.), botulinum neurotoxin type A + TSP11 (BoNT/A + TSP11), and botulinum neurotoxin type A + TSP23 (BoNT/A + TSP23) to the right gastrocnemius muscle of the mice described in Example 9. CMAP values were analyzed by using a two-tailed t-test (**p<0.01, ***p<0.001, and ns; not significant).

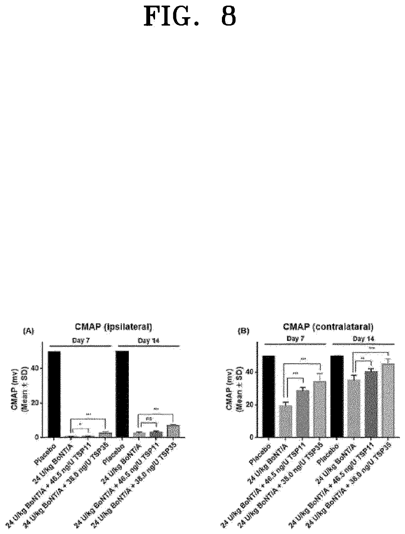

FIG. 8 is a graph showing CMAP values of a muscle of the administered site (A) and a contralateral muscle of the non-administered site (B) on day 7 and day 14, after administering placebo, botulinum neurotoxin type A (BoNT/A, Coretox inj.), botulinum neurotoxin type A + TSP11 (BoNT/A + TSP11), and botulinum neurotoxin type A + TSP35 (BoNT/A + TSP35) to the right gastrocnemius muscle of the mice described in Example 10. CMAP values were analyzed by using a two-tailed t-test (*p<0.05, **p<0.01, ***p<0.001, and ns; not significant).

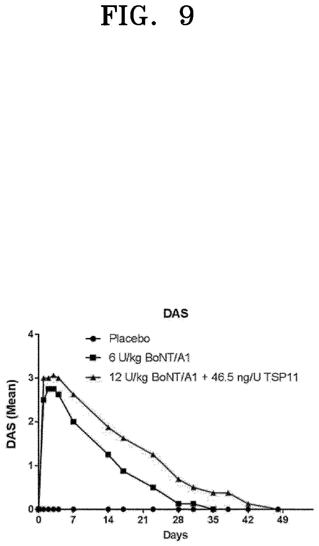

FIG. 9 is a graph showing average DAS values over time after administering placebo, 6 U/kg botulinum neurotoxin type A (BoNT/A), and 12 U/kg botulinum neurotoxin type A + TSP11 (BoNT/A + TSP11) to the right gastrocnemius muscle of mice described in Example 11.

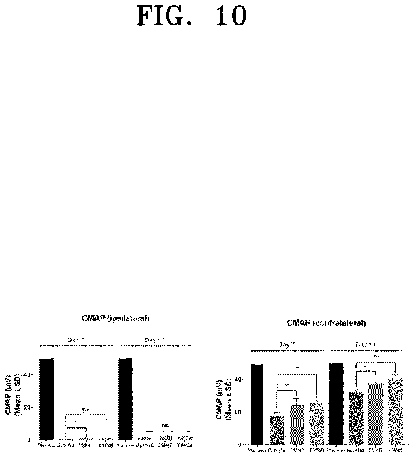

FIG. 10 is a graph showing CMAP values of a muscle of the administered site (A) and a contralateral muscle of the non-administered site (B) on day 7 and day 14, after administering placebo, botulinum neurotoxin type A (BoNT/A, Coretox inj.), botulinum neurotoxin type A + TSP47 (BoNT/A + TSP47), and botulinum neurotoxin type A + TSP48 (BoNT/A + TSP48) to the right gastrocnemius muscle of the mice described in Example 12. CMAP values were analyzed by using a two-tailed t-test (*p<0.05, **p<0.01, ***p<0.001, and ns; not significant).

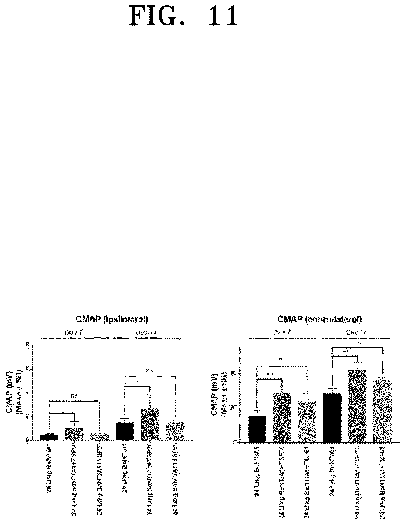

FIG. 11 is a graph showing CMAP values of a muscle of the administered site (A) and a contralateral muscle of the non-administered site (B) on day 7 and day 14, after administering botulinum neurotoxin type A (BoNT/A, Coretox inj.), botulinum neurotoxin type A + TSP56 (BoNT/A + TSP56), and botulinum neurotoxin type A + TSP61 (BoNT/A + TSP61) to the right gastrocnemius muscle of the mice described in Example 13. CMAP values were analyzed by using a two-tailed t-test (*p<0.05, **p<0.01, ***p<0.001, and ns; not significant).

### Mode for the Invention

As used herein, the term "botulinum toxin (BoNT)" may be any polypeptides or fragments of botulinum toxins. In an embodiment, a botulinum toxin may refer to a botulinum toxin derivative, i.e., a compound having botulinum toxin activity but containing one or more chemical or functional alterations on any part or on any chain of naturally occurring or recombinant native botulinum toxins. For example, the botulinum toxin may be a modified neurotoxin having one or more amino acids deleted, modified, or substituted compared to native forms thereof, or the modified neurotoxin may be a neurotoxin or a derivative or fragment thereof produced by recombination techniques.

The botulinum toxin may be a botulinum toxin (botulinum toxin protein molecule with about 150 kD belonging to botulinum toxin serotypes A to G) linked or not linked to an endogenous non-toxin protein (hemagglutinin protein and non-toxin non-hemagglutinin protein produced by *Clostridium botulinum*).

Botulinum toxins are classified into 7 serotypes (botulinum toxin type A, botulinum toxin type B, botulinum toxin type C, botulinum toxin type D, botulinum toxin type E, botulinum toxin type F, and botulinum toxin type G), which are further subdivided into subtypes based on variations in amino acid sequence. The different serotypes of botulinum toxin vary in the animal species affected thereby and in the severity and duration of paralysis caused thereby.

The term "amino acid" refers to not only naturally occurring amino acids and non-naturally occurring amino acids, but also amino acids such as proline, amino acid analogs, and amino acid mimics that function in a similar manner to that of naturally occurring amino acids. That is, the amino acid mimic refers to functioning in a similar manner to that of naturally occurring amino acids while having different structures from general chemical structures of the naturally occurring amino acids.

The terms "polypeptide" and "peptide" are used interchangeably and refer to polymers formed of amino acid residues.

The term "identity" refers to relevance between sequences of two or more polypeptides or polynucleotides determined by comparing the sequences. The identity represents a degree of sequence relatedness determined by the number of matches between strings of two or more amino acid residues or nucleotide residues. The identity between related polypeptides or polynucleotides may be calculated by any known method. "% identity" applied to polypeptides and polynucleotides is defined as a percentage of residues of a candidate amino acid sequence or nucleotide sequence identical to residues of a second sequence, after aligning, if necessary, introducing gaps, to obtain a maximum percent identity with the second sequence. Methods and programs for the alignment are known in the art. The program may be, for example, BLAST, Smith-Waterman algorithm, or Needleman-Wunsch algorithm.

An aspect provides a composition for stabilizing a botulinum neurotoxin including a polypeptide inhibiting diffusion of the botulinum neurotoxin *in vivo* and represented by Formula 1 below.

[Formula 1] (C)ₙ-V

In the formula, C represents a moiety interacting with a cell membrane of a nerve cell, V represents a moiety interacting with a cell membrane of a nerve cell and interacting with the botulinum neurotoxin, and n is an integer of 0 or 1.

In an embodiment, V may be a VAMP (vesicle associated membrane protein), a BDNF (brain-derived neurotrophic factor), a fragment thereof, or a variant thereof.

In an embodiment, V may be VpN of VAMP, a fragment thereof, or a variant thereof.

In an embodiment, VAMP may be VAMP1, VAMP2, or VAMP3, for example, VAMP2.

For example, V may be VAMP2, a fragment thereof, or a variant thereof. For example, V may be VpN of VAMP2, a fragment thereof, or a variant thereof.

In an embodiment, V may comprise the amino acid sequence of SEQ ID NO: 1.

In an embodiment, V may consist of the amino acid sequence of SEQ ID NO: 1, 2, 3, 4, 5, 6, 7, 8, 9, or 10; or the amino acid sequence SEQ ID NO: 1, 2, 3, 4, 5, 6, 7, or 8 of which one amino acid is substituted. The amino acid of SEQ ID NO: 8 is VpN, amino acids of SEQ ID NOS: 1 to 7 are fragments of VpN, and amino acids of SEQ ID NOS: 9 and 10 are variants of VpN.

V may be selected from: amino acid sequences of SEQ ID NOS: 1, 2, 3, 4, 5, 6, 7, and 8; and amino acid sequences of SEQ ID NO: 1, 2, 3, 4, 5, 6, 7, and 8 of which one amino acid is substituted.

The amino acid sequence of SEQ ID NO: 1, 2, 3, 4, 5, 6, 7, or 8, of which one amino acid is substituted, may have reduced hydrophobic interaction compared to the sequence before substitution. For example, it may be the amino acid sequence of SEQ ID NO: 1, 2, 3, 4, 5, 6, 7, or 8, methionine and/or threonine of which is substituted with alanine. For example, it may be M47A or T36A variation of the amino acid sequence of SEQ ID NO: 8. In an embodiment, C may be a cell penetrating peptide or a cationic peptide.

For example, a cationic amino acid may be selected from lysine, arginine, and histidine. For example, the cationic amino acid may be arginine.

In an embodiment, C may be an arginine-rich peptide. For example, in C, 3/5 or more or 2/3 or more of the total amino acids may be composed of arginine.

In an embodiment, C may further include an aromatic amino acid.

For example, the aromatic amino acid may be selected from tryptophan, phenylalanine, and tyrosine.

In an embodiment, the aromatic amino acid may be tryptophan.

In an embodiment, C may include 6 to 9 arginines and 2 to 3 of tryptophans. For example, C may consist of 6 to 9 arginines and 2 to 3 tryptophans.

For example, in the case where C consists of 6 to 9 arginines and 2 to 3 tryptophans, a same sequence of amino acids may be repeated up to three times in C.

In an embodiment, C may have a three-dimensional structure with arginine and tryptophan biased toward one side.

In an embodiment, C may consist of an amino acid sequence of SEQ ID NO: 11, SEQ ID NO: 12, or SEQ ID NO: 13.

In an embodiment, the polypeptide may consist of 10 to 50, 10 to 40, 15 to 40, or 15 to 35 amino acids, for example 17 to 32 or 25 to 32 amino acids, for example 17, 25, 26, 31, or 32 amino acids.

In an embodiment, the polypeptide may consist of the amino acid sequence of SEQ ID NO: 8 (TSP19), SEQ ID NO: 14 (TSP11), SEQ ID NO: 15 (TSP28), SEQ ID NO: 16 (TSP17), SEQ ID NO: 17 (TSP35), SEQ ID NO: 18 (TSP23), SEQ ID NO: 19 (TSP2), SEQ ID NO: 20 (TSP56), or SEQ ID NO: 21 (TSP61).

The polypeptide may be a variant of the disclosed amino acid sequences. The variant may be a polypeptide having a sequence identity of 80 % or more with the disclosed amino acid sequence. For example, the variant may have a sequence identity of 82.5 % or more, 85 % or more, 90 % or more, 95 % or more, or 98% or more with the disclosed amino acid sequence.

In the polypeptide, the N-terminus may be protected by a protective group. The N-terminus may bind to an acetyl group, a fluoreonylmethoxycarbonyl group, a formyl group, a palmitoyl group, a myristyl group, a stearyl group, a butoxycarbonyl group, an allyloxycarbonyl group, or a polyethylene glycol (PEG), as the protective group.

The C-terminus of the polypeptide may be protected by a protective group. The C-terminus may bind to an amino group (-NH₂), a tertiary alkyl group, or an azide group (-NHNH₂), as the protective group. In the tertiary alkyl group, the alkyl group may include 4 to 20, 4 to 16, 4 to 12, 4 to 8, 5 to 20, 5 to 16, 5 to 12, or 5 to 8 carbon atoms.

Another aspect provides a method of inhibiting diffusion of a botulinum neurotoxin *in vivo* by adding a polypeptide of Formula 1 to the botulinum neurotoxin or a formulation including the same.

Another aspect provides a method of stabilizing a botulinum neurotoxin by adding the polypeptide of Formula 1 to the botulinum neurotoxin or a formulation including the same.

Another aspect provides a use of the polypeptide of Formula 1 for inhibiting diffusion of a botulinum neurotoxin *in vivo.*

Another aspect provides a use of the polypeptide of Formula 1 for stabilizing a botulinum neurotoxin.

Another aspect provides a use of the polypeptide of Formula 1 as a botulinum neurotoxin excipient.

Another aspect provides a botulinum neurotoxin formulation including a botulinum neurotoxin and the composition for stabilizing the botulinum neurotoxin.

Another aspect provides the polypeptide of Formula 1 for inhibiting diffusion of a botulinum neurotoxin *in vivo.*

Another aspect provides the polypeptide of Formula 1 for stabilizing a botulinum neurotoxin.

Another aspect provides the polypeptide of Formula 1 for use as a botulinum neurotoxin excipient.

In an embodiment, the botulinum neurotoxin may be type A, B, C, D, E, F, or G.

In an embodiment, the botulinum neurotoxin may be a naturally occurring or a recombinant toxin.

In an embodiment, the recombinant toxin may be disclosed in Korean Patent Application No. 10-2021-0083930 (the disclosure of which is incorporated herein by reference in its entirety), and the recombinant toxin may include a recombinant botulinum toxin type A light chain in which a sequence of a third domain of first, second, third, and fourth domains of a botulinum toxin type A light chain other than botulinum toxin type A4 is substituted with a sequence of a third domain of botulinum toxin type A4 or a variant thereof. For example, a sequence of the fourth domain of the first, second, third, and fourth domains of the recombinant botulinum toxin type A light chain may further be substituted with a fourth domain of the botulinum toxin type A4 or a variant thereof. For example, the botulinum toxin type A light chain may be botulinum toxin type A1, A2, A3, A5, A6, A7, or A8, for example, botulinum toxin type A1. For example, the recombinant botulinum toxin type A light chain may include A1-A1-A4-A4 as the first, second, third, and fourth domains. For example, the recombinant botulinum toxin type A light chain may consist of the amino acid sequence of SEQ ID NO: 23.

In an embodiment, the recombinant toxin may be disclosed in Korean Patent Application No. 10-2021-0083950 (the disclosure of which is incorporated herein by reference in its entirety), and the recombinant toxin may include a recombinant botulinum toxin type A light chain in which a sequence of a second domain of first, second, third, and fourth domains of a botulinum toxin type A light chain other than botulinum toxin type A1 is substituted with a sequence of a second domain of botulinum toxin type A1 or a variant thereof. For example, a sequence of the fourth domain of the first, second, third, and fourth domains of the recombinant botulinum toxin type A light chain may further be substituted with a fourth domain of the botulinum toxin type A1 or a variant thereof. For example, the botulinum toxin type A light chain may be botulinum toxin type A2, A3, A4, A5, A6, A7, or A8, for example, botulinum toxin type A4. For example, the recombinant botulinum toxin type A light chain may include A4-A1-A4-A1 as the first, second, third, and fourth domains.

In an embodiment, the botulinum neurotoxin may be in the form of 7S (150kD) or 19S (900kD).

In an embodiment, an amount of the polypeptide used is not particularly limited, as long as the polypeptide exhibits activity that inhibits diffusion of a botulinum neurotoxin without inhibiting the intended clinical efficacy of the botulinum neurotoxin, but may be, for example, 10 to 10¹⁰ times, 10² to 10⁹ times, 10⁸ to 10⁸ times, 10³ to 10⁷ times, 10³ to 10⁸ times, or 3×10³ to 10⁶ times as much as 1 mol of the botulinum neurotoxin.

The botulinum neurotoxin formulation may further include a pharmaceutically acceptable excipient or carrier.

The term "pharmaceutically acceptable" means that the composition for stabilizing a botulinum neurotoxin or ingredients thereof are suitable for use in contact with tissues or for use in patients without excessive toxicity, instability, allergic response, and the like. If pharmaceutically or cosmetically typical and acceptable, the botulinum neurotoxin formulation of the present disclosure may include any ingredient commonly used in the pharmaceutical and dermatological fields.

The botulinum neurotoxin formulation of the present disclosure may be prepared by mixing with one or more additional pharmaceutically acceptable excipients or additives. For example, it may include a simple aqueous pharmaceutically acceptable excipient or additive such as buffered saline. In this case, the botulinum neurotoxin formulation of the present disclosure may be applied to injection. The pharmaceutically acceptable excipient or additive may be a stabilizer, an ionic compound, a surfactant, a buffer, a lyophilization protectant, or a combination thereof, for example, an amino acid (e.g., methionine), a salt (e.g., NaCl), a buffer solution, a non-ionic surfactant (e.g., polysorbate, such as polysorbate 20), sugar (e.g., disaccharide such as white sugar), sugar alcohol (e.g., sorbitol), or a combination thereof

The botulinum neurotoxin formulation may not include albumin or animal-derived ingredients or polysaccharides.

The botulinum neurotoxin formulation may be formulated in any form, such as a solid or liquid preparation, for example, a lyophilized powder, a liquid phase, or a prefilled syringe preparation.

The botulinum neurotoxin formulation of the present disclosure may include solutions, emulsions (including microemulsions), suspensions, creams, lotions, gels, powders, or other typical solid or liquid compositions suitable for application to the skin or other tissues to which the botulinum neurotoxin formulation of the present disclosure is applicable. Such compositions may include, in addition to the botulinum neurotoxin and the stabilizing peptide, commonly used other ingredients, such as antimicrobial agents, moisturizers, hydrating agents, penetration agents, preservatives, emulsifiers, natural or synthetic oils, solvents, surfactants, detergents, gellants, emollients, anti-oxidants, aromatic agents, fillers, thickeners, wax, odor absorbers, dyes, colorants, powders, viscosity modifiers, and water, and optionally, anesthetics, anti-itch actives, phytoextracts, conditioning agents, darkening agents, or lightening agents, glitters, humectants, mica, minerals, polyphenol, siliton or derivatives, sunblock, vitamins, and phytomedicinals.

Another aspect provides a polypeptide consisting of the amino acid sequence of SEQ ID NO: 15, SEQ ID NO: 16, SEQ ID NO: 17, or SEQ ID NO: 18 for use in the composition for stabilizing a botulinum neurotoxin and the formulation.

Another aspect provides a use for amelioration or treatment of a disease by administering the botulinum neurotoxin formulation to an individual.

The "administration" or "administering" refers to a process of introducing a pharmaceutical composition or an active ingredient into a subject. The pharmaceutical composition may be administered via various suitable routes.

The "pharmaceutical composition" refers to a formulation in which an active ingredient is a botulinum neurotoxin. The term "formulation" means that at least one additional ingredient, in addition to a botulinum neurotoxin active ingredient, for example, albumin human (human serum albumin or recombinant human albumin) and/or sodium chloride, is present in the pharmaceutical composition. The pharmaceutical composition is a formulation suitable for administration to a subject such as a human patient. The pharmaceutical composition may be in the form of a lyophilized form, for example, a solution formed after reconstitution of a lyophilized pharmaceutical composition using saline or water, or a solution that does not require reconstitution. The pharmaceutical composition may be a liquid or solid.

The present disclosure is applied to administer an effective amount of a botulinum neurotoxin formulation. As used herein, the term "effective amount" refers to an amount of a botulinum neurotoxin formulation sufficient to obtain a desired effect, but safe, i.e., to the extent that serious side effects are prevented. The desired effect includes, for example, relaxation of specific muscles for the purpose of reducing fine wrinkles, particularly, fine wrinkles of a face, or adjusting the appearance of a face, or generally relieving muscle tension. The general relief of muscle tension may occur in the face or other regions.

The "treat", "treating", or "treatment" refers to alleviation or reduction (including partial reduction, substantial reduction, and almost complete reduction) of a disease, disorder, or abnormality, or (temporary or permanent) resolution or prevention thereof, to obtain a desired therapeutic result, for example, by healing injured or damaged tissue, or altering, modifying, reinforcing, alleviating, ameliorating, and/or beautifying the existing or recognized disease, disorder, or abnormality. Throughout the specification, the "treatment" is a concept including prevention. The "prevention" refers to delaying the onset of a disease, disorder, or illness. If the onset of a disease, disorder, or illness is delayed for a preset period of time, prevention may be considered complete.

Another aspect provides a use for alleviating or treating a disease by administering the botulinum neurotoxin formulation to an individual.

The "individual" refers to a subject in need of treatment of a disease. The individual may be a mammal such as human, mouse, cat, horse, and cow.

The present disclosure is provided to alleviate or treat a disease by administering the botulinum neurotoxin formulation to a patient. In an embodiment, the stabilized composition for stabilizing a botulinum neurotoxin may be applied to an individual or patient in need of treatment of a disease in an effective amount, for example, to prevent or treat muscle paralysis, to reduce hypersecretion or sweating, to treat neurological pain or migraines, to reduce muscle spasms, to prevent or alleviate acne, to alleviate or enhancing immune response, to reduce wrinkles, or to prevent or treat various other diseases. In an embodiment, the botulinum neurotoxin formulation is administered by parenteral injection such as subcutaneous injection. The administration may be conducted, for example, via leg, shoulder, waist, palm, foot, neck, groin, dorsum of hand, dorsum of foot, elbow, upper arm, knee, upper limb, buttock, body, pelvis, or any body part suitable for administration of the botulinum neurotoxin composition.

The present disclosure also includes a transdermal delivery device for delivering the composition for stabilizing a botulinum neurotoxin disclosed in this specification via the skin. Such a device may have a simple structure such as a skin patch, or may be a device including a component for dispensing the composition and monitoring the dispensing of the composition, and a component for monitoring individual's response to the distributed pharmaceutical composition.

The botulinum neurotoxin formulation of the present disclosure may have a pH suitable for use in a physiological environment having a pH range of about 4.5 to about 6.3. The botulinum neurotoxin formulation according to the present disclosure may be stored at room temperature or under refrigerated conditions.

A therapeutically effective amount of the botulinum neurotoxin is about 0.01 U/kg to about 100 U/kg, about 0.1 U/kg to about 100 U/kg, about 0.2 U/kg to about 100 U/kg, about 0.2 U/kg to about 50 U/kg, about 0.2 U/kg to about 30 U/kg, about 0.2 U/kg to about 10 U/kg, or about 0.2 U/kg to about 1 U/kg. In another embodiment, based on an adult with a weight of 60 kg, about 1 U to about 10,000 U, about 1 U to about 5,000 U, about 1 U to about 2,500 U, about 1 U to about 1,000 U, about 1 U to about 500 U, about 1 U to about 300 U, about 1 U to about 200 U, about 10 U to about 200 U, about 10 U to about 100 U, or about 10 U to about 50 U may be used.

As used herein, the term "unit(s)" or "U" refers to LD₅₀ dose, defined as the amount of a botulinum neurotoxin that kills 50% of mice injected with the botulinum neurotoxin, and may be interchangeably used.

Another aspect provides a method of stabilizing a botulinum neurotoxin by administering the botulinum neurotoxin formulation to an individual.

The term "stabilization" refers to minimizing formation of (insoluble and/or soluble) aggregates or chemical degradation during storage or production of a pharmaceutical composition and maintaining the pH and intrinsic structure of a protein to maintain substantial retention of biological activity and protein stability. In addition, it also refers to reducing the risk of side effects by inhibiting diffusion of the botulinum neurotoxin to other tissues.

In the case where the botulinum neurotoxin is topically applied to the body for the purpose of alleviating or treating a disease, the botulinum neurotoxin may diffuse over time, causing unwanted muscle paralysis in other parts of the body. For example, in the case where the botulinum neurotoxin is administered to areas around eyes to reduce wrinkles and diffuses into adjacent tissues, "Ptosis" may be caused, thereby interfering with normal vision.

After administering the botulinum neurotoxin formulation of the present disclosure to a human body, diffusion thereof may be reduced. Therefore, the botulinum neurotoxin is accurately delivered to a target tissue, and thus unwanted side effects related to the diffusion of the botulinum neurotoxin may be reduced.

Because unwanted side effects associated with diffusion of the botulinum neurotoxin are reduced, the botulinum neurotoxin formulation of the present disclosure may include an increased amount of the botulinum neurotoxin compared to the amount commonly used in formulations (used in formulations not including the polypeptide). In this case, an increased duration of clinical efficacy of the botulinum neurotoxin, compared to conventional botulinum neurotoxin formulations, may be obtained. Accordingly, the administration cycle may be increased and the administration frequency may be decreased. In an embodiment, the botulinum neurotoxin formulation may include the botulinum neurotoxin in an amount 1.5 times, 2 times, 3 times, 4 times, or 5 times or more than commonly used amounts. In an embodiment, the botulinum neurotoxin formulation may be administered with a 1.5 times, 2 times, 3 times, or 4 times or longer administration cycle, for example, with a cycle of longer than 3 month, 4 months, 5 months, 6 months, 7 months, 8 months, 9 months, 10 months, 11 months, or 12 months or more.

Hereinafter, the present disclosure will be described in more detail with reference to the following examples. However, the following examples are merely presented by exemplify the present disclosure and is not intended to limit the scope of the disclosure in any way.

In this experiment, polypeptides shown in Table 1 below were synthesized (Company: ANYGEN) and used. The structures of Table 1 below also show whether an amino group is bonded as a protective group in addition of the amino acid structures according to SEQ ID NO:.

**[Table 1]**

| Name of peptide | SEQ ID NO: | Structure | Remarks |
|---|---|---|---|
| TSP1 | SEQ ID NO: 22 | RKKRRQRRRGKKKKKKKKKK KKKKKGRKKRRQRRR-NH2 | |
| TSP2 | SEQ ID NO: 19 | RALTMDSKKRIGWRFIRIDTS CVCTLTIKRGR-NH2 | derived from BDNF |
| TSP11 | SEQ ID NO: 14 | RRWRRWNRFNRRRCRRRLQ QTQAQVDEVVDIM | IMTP8+VPN |
| TSP17 | SEQ ID NO: 16 | RRWRRWNRFNRRRCRRRLQ QTQAQV | IMTP8+VPN fragment |
| TSP19 | SEQ ID NO: 8 | RRLQQTQAQVDEVVDIM-NH2 | VPN alone |
| TSP23 | SEQ ID NO: 18 | RRWRRWNRFNRRRCRGWR FIRIDTSCVCTLT-NH2 | IMTP8 + TSP2(BDNF) |
| TSP28 | SEQ ID NO: 15 | RRWWRRWRRRRLQQTQAQ VDEVVDIM-NH2 | In TSP11 sequence, sequence of C moiety is substituted with 6 arginines and 3 tryptophans |
| TSP35 | SEQ ID NO: 17 | RRRWRRWRRRRLQQTQAQV DEVVDIM-NH2 | In TSP28 sequence, one W sequence is substituted with R |
| TSP47 | SEQ ID NO: 14 | RRWRRWNRFNRRRCRRRLQ QTQAQVDEVVDIM-NH2 | In TSP11 sequence, C-term amidation |
| TSP48 | SEQ ID NO: 15 | RRWWRRWRRRRLQQTQAQ VDEVVDIM | In TSP28 sequence, no amidation |
| TSP56 | SEQ ID NO: 20 | RRWRRWNRFNRRRCRRRLQ QTQAQVDEVVDIA | In TSP11 sequence, 1 amino acid of VPN is replaced (M47A) |
| TSP61 | SEQ ID NO: 21 | RRWRRWNRFNRRRCRRRLQ QAQAQVDEVVDIM | In TSP11 sequence, 1 amino acid of VPN is replaced (T36A) |

### Example 1: Non-clinical Efficacy Test of Mixed Formulation of Botulinum Neurotoxin Type A and TSP11 in Mice

5-week-old female CD1 (ICR) mice were purchased from Orient Bio Co., Ltd., and after acclimatization and quarantine for 1 week, 6-week-old mice were used in the experiment. The feed was supplied ad libitum with sterilized solid feed for laboratory animals (R40-10, SAFE, France), and drinking water was supplied ad libitum with autoclaved tap water. During the acclimation, quarantine, and experimental period, the mice were housed under specific-pathogen-free conditions set at a temperature of 23 ± 3 °C, relative humidity of 55 ± 15 %, illumination time of 12 hours (8:00 a.m. to 8:00 p.m.), ventilation frequency of 15 times/hour, and illumination intensity of 150 to 300 Lux. This study was conducted after review and approval by the Medytox Animal Experiment Ethics Committee (A-2020-016).

Coretox inj. (Medytox) was used as a test substance, and a sterilized saline solution (Daehan Pharmaceutical Industry) was used as placebo (placebo-administered group. A toxin-stabilizing peptide (TSP11) was mixed with a botulinum neurotoxin in a ratio of 3×10⁴ to 1×10⁵ per 1 mol of the botulinum neurotoxin and used in the experiment (Table 2). The mixture of the botulinum neurotoxin and the toxin-stabilizing peptide was diluted to a concentration of 120 U/mL and used in the experiment.

The day the test substance was administered was set as day 0. After anesthetizing the mice using an injection anesthetic (100 mg/kg ketamine hydrochloride + 10 mg/kg xylazine), each test substance was administered at 0.2 mL/kg to the right gastrocnemius muscle of the mice using a Hamilton syringe according to the compositions of the groups shown in Table 2.

**[Table 2]**

| Group | No. of animals | Administered substance and dose | Route of administration | Evaluation indicator |
|---|---|---|---|---|
| 1 | 6 | Placebo | Right gastrocnemius muscle | CMAP |
| 2 | 6 | 24 U/kg BoNT/A | Right gastrocnemius muscle | CMAP |
| 3 | 6 | 24 U/kg BoNT/A + 15.5 ng/U TSP11 | Right gastrocnemius muscle | CMAP |
| 4 | 6 | 24 U/kg BoNT/A + 4.65 ng/U TSP11 | Right gastrocnemius muscle | CMAP |

For evaluation, the compound muscle action potential (CMAP) test method, which measures the action potential of muscles responding to external electrical stimulation, was used. CMAP was measured by using a Nicolet Viking Quest (Viasys Healthcare, Inc.) or a UltraPro S100 (Natus Neurology Inc.) in the right gastrocnemius muscle region (administered site) and a left gastrocnemius muscle (non-administered site) of each mouse. After anesthesia with an injection anesthetic, fur was removed from the measurement area and the mouse was placed in a prone position. A negative electrode was placed on the sciatic nerve area on the side of the leg to be measured, and a positive electrode was placed approximately 1 cm away from the area based on the spine. A recording electrode and a reference electrode were placed on the gastrocnemius muscle region and the Achilles tendon region, respectively, and the ground electrode was placed on the rectus femoris region. The level and duration of stimulation were 7 to 8 mA, 0.1 ms, and a filter range of an amplifier was set to 2 to 10 K at 60 Hz. When measured under the above-described conditions, a height from the base of a waveform to the peak was converted into CMAP measurement data. On day 7 and day 14 after administering the test substance, measurement was performed at the gastrocnemius muscle of the administered site and at the gastrocnemius muscle of the contralateral leg.

Graphpad Prism 7.05 (GraphPad Software Inc., CA, USA) was used for graph presentation, and SPSS software 25.0 (SPSS Inc., IL, USA) and Excel (2013, MS, USA) were used for statistical analysis. The result of the experiment was expressed as mean ± standard deviation. Normality was tested using the Kolmogorov-Smirnov test. Non-parametric data were statistically analyzed using the Mann-Whitney test, and for parametric data, a two-tailed t-test was conducted, and a *p*-value less than 0.05 was judged to be statistically significant.

After administering the botulinum neurotoxin type A (BoNT/A) and the BoNT/A + TSP11 test substance, in which the toxin-stabilizing peptide was mixed, to the right gastrocnemius muscle of the mice at a dose of 24 U/kg, CMAP values were measured at a muscle of the administered site and a contralateral muscle of the non-administered site on day 7 and day 14. As a result, although a significant difference was not observed in the CMAP values of the muscles at the administered site between the BoNT/A alone-administered group and the BoNT/A + TSP11-administered group, the CMAP values of the contralateral muscle of the BoNT/A + 15.5 ng/U TSP11-administered group and the BoNT/A + 4.65 ng/U TSP11-administered group were observed as significantly higher values than that of the BoNT/A alone-administered group (FIG. 1). Based on these results, it was confirmed that the effect on reducing diffusion to the contralateral leg was obtained by mixing the botulinum neurotoxin and TSP11.

### Example 2: Non-clinical Efficacy Test of Mixed Formulation of Recombinant Botulinum Neurotoxin Type A and TSP11 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1. This study was conducted after review and approval by the Medytox Animal Experiment Ethics Committee (A-2020-016).

A sterilized saline solution (Daehan Pharmaceutical Industry) was used as placebo (placebo-administered group). The recombinant botulinum neurotoxin type A (eBoNT/A), as a recombinant botulinum neurotoxin including a combination of BoNT/A1 light chains and BoNT/A4 light chains as light chains of the botulinum neurotoxin, was produced by cloning a recombinant full-length botulinum neurotoxin gene (SEQ ID NO: 24) using a pMTL80000 vector system by the infusion-HD (Takara) by using a detoxified hall A-hyper strain in which the toxin gene was inactivated, and purified into about 150 kDa form that does not include complex components. All manufacturing of the above recombinant botulinum neurotoxin was performed in a facility licensed for toxin production according to government regulations. A toxin-stabilizing peptide (TSP11) was mixed in a ratio of 4.65 to 46.5 ng/U and used in the experiment (Table 3).

The administered dose of each test substance was administered at the same dose based on unit (U: a value representing the biological activity of a botulinum neurotoxin, where 1 U is the median lethal dose for mice administered therewith by intraperitoneal injection). The mixture of the botulinum neurotoxin and the toxin-stabilizing peptide was diluted to a concentration of 60 U/mL and used in the experiment.

The day the test substance was administered was set as day 0, After anesthetizing the mice using an injection anesthetic (100 mg/kg ketamine hydrochloride + 10 mg/kg xylazine), each test substance was administered to the right gastrocnemius muscle of the mice at 0.2 mL/kg using a Hamilton syringe according to the compositions of the groups shown in Table 3. 6 mice were used for each test substance in one test, and the test was repeated twice to three times for each test substance. For evaluation, the compound muscle action potential (CMAP) test method, which measures the action potential of muscles responding to external electrical stimulation, was used. Specific evaluation methods and statistical analysis were conducted in the same manner as in Example 1.

**[Table 3]**

| Grou p | No. of animals | Administered substance and dose | Route of administration | Evaluation indicator |
|---|---|---|---|---|
| 1 | 18 | Placebo | Right gastrocnemius muscle | CMAP |
| 2 | 18 | 12 U/kg eBoNT/A | Right gastrocnemius muscle | CMAP |
| 3 | 12 | 12 U/kg eBoNT/A + 4.65 ng/U TSP11 | Right gastrocnemius muscle | CMAP |
| 4 | 18 | 12 U/kg eBoNT/A + 15.5 ng/U TSP11 | Right gastrocnemius muscle | CMAP |
| 5 | 18 | 12 U/kg eBoNT/A + 46.5 ng/U TSP11 | Right gastrocnemius muscle | CMAP |

After administering the recombinant botulinum neurotoxin type A (eBoNT/A) test substance and the eBoNT/A + TSP11 test substance, in which the recombinant botulinum neurotoxin type A was mixed with the toxin-stabilizing peptide, to the right gastrocnemius muscle of the mice at a dose of 12 U/kg, CMAP values were measured at a muscle of the administered site and a contralateral muscle of the non-administered site on day 7 and day 14. As a result, although a significant increase in the CMAP value was observed at the muscle of the administered site on day 7 in the eBoNT/A + 46.5 ng/U TSP11-administered group compared to the eBoNT/A alone-administered group, but was not observed in the test groups administered with the mixture of the toxin-stabilizing peptide on day 14 compared to the eBoNT/A alone-administered group (FIG. 2). Significant increases in the CMAP values were observed at the contralateral muscles on both day 7 and day 14 in the eBoNT/A + TSP11-administered group compared to the BoNT/A-administered group (FIG. 2). Based on these results, it was confirmed that diffusion of the recombinant botulinum neurotoxin type A to the contralateral leg was reduced by mixing with the toxin-stabilizing peptide (TSP11).

### Example 3: IM LD₅₀ Test of Mixed Formulation of Botulinum Neurotoxin Type A and TSP1, TSP11, or TSP28 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1. This study was conducted after review and approval by the Medytox Animal Experiment Ethics Committee (A-2020-015).

Coretox inj. (Medytox) was used as a test substance, and the toxin-stabilizing peptides (TSP1, TSP11, and TSP28) were mixed with the botulinum neurotoxin in a ratio of 3×10⁵ per 1 mol of the botulinum neurotoxin and used in the experiment (Table 4). The mixture of the botulinum neurotoxin and the toxin-stabilizing peptide was diluted to concentrations of 300, 450, 675, and 1012.5 U/mL and used in the experiment.

The day the test substance was administered was set as day 0, and anesthesia of the test animals and administration of the test substance were performed in the same manner as in Example 1. 10 mice were used for each test group in one test, and the test was repeated twice in total. After administering the test substance to the right gastrocnemius muscle, dead individuals were identified for 7 days. For dead individuals per each administered dose, IM LD₅₀ values were calculated by PROBIT analysis by using SPSS software 25.0 (SPSS Inc., IL, USA).

**[Table 4]**

| Group | No. of animals | Administered substance and dose | Route of administration | Evaluation indicator |
|---|---|---|---|---|
| 1 | 10 | 30 U/kg BoNT/A | Right gastrocnemius muscle | Survival |
| 2 | 10 | 90 U/kg BoNT/A | Right gastrocnemius muscle | Survival |
| 3 | 10 | 135 U/kg BoNT/A | Right gastrocnemius muscle | Survival |
| 4 | 10 | 30 U/kg BoNT/A + 50.9 ng/U TSP1 | Right gastrocnemius muscle | Survival |
| 5 | 10 | 90 U/kg BoNT/A + 50.9 ng/U TSP1 | Right gastrocnemius muscle | Survival |
| 6 | 10 | 135 U/kg BoNT/A + 50.9 ng/U TSP1 | Right gastrocnemius muscle | Survival |
| 7 | 10 | 202.5 U/kg BoNT/A + 50.9 ng/U TSP1 | Right gastrocnemius muscle | Survival |
| 8 | 10 | 30 U/kg BoNT/A + 38.3 ng/U TSP28 | Right gastrocnemius muscle | Survival |
| 9 | 10 | 90 U/kg BoNT/A + 38.3 ng/U TSP28 | Right gastrocnemius muscle | Survival |
| 10 | 10 | 135 U/kg BoNT/A + 38.3 ng/U TSP28 | Right gastrocnemius muscle | Survival |
| 11 | 10 | 202.5 U/kg BoNT/A + 38.3 ng/U TSP28 | Right gastrocnemius muscle | Survival |
| 12 | 10 | 30 U/kg BoNT/A + 46.5 ng/U TSP11 | Right gastrocnemius muscle | Survival |
| 13 | 10 | 90 U/kg BoNT/A + 46.5 ng/U TSP11 | Right gastrocnemius muscle | Survival |
| 14 | 10 | 135 U/kg BoNT/A + 46.5 ng/U TSP11 | Right gastrocnemius muscle | Survival |
| 15 | 10 | 202.5 U/kg BoNT/A + 46.5 ng/U TSP11 | Right gastrocnemius muscle | Survival |

**[Table 5]**

| Test substance | Median lethal dose by intramuscular administration once (U/kg) | Median lethal dose by intramuscular administration twice (U/kg) | Median lethal dose by intramuscular administration once to twice on average (U/kg) |
|---|---|---|---|
| BoNT/A | 98.8 | 83.3 | 91.1 |
| BoNT/A + TSP1 | 81.2 | 71.9 | 76.6 |
| BoNT/A + TSP28 | 128.5 | 122.1 | 125.3 |
| BoNT/A + TSP11 | 152.1 | 142.8 | 147.5 |

As a result of calculating the median lethal dose (IM LD₅₀) by observing dead individuals for 7 days after administering the botulinum neurotoxin type A (BoNT/A) and the BoNT/A + TSP1, BoNT/A + TSP28, and BoNT/A + TSP11 test substances, in which the toxin-stabilizing peptides were mixed, to the right gastrocnemius muscle of the mouse as shown in Table 5, the median lethal doses (IM LD₅₀) were observed as 91.1 U/kg on average in the BoNT/A alone-administered group, as 76.6 U/kg on average in the BoNT/A + TSP1-administered group, 125.3 U/kg on average in the BoNT/A + TSP28-administered group, and 147.5 U/kg on average in the BoNT/A + TSP11-administered group. About a 1.4-fold increase was observed in the BoNT/A + TSP28-administered group, and about a 1.6-fold increase was observed in the BoNT/A + TSP11-administered group compared to the BoNT/A alone-administered group (Table 5). Based on these results, the safety-improving effect by mixing the botulinum neurotoxin and the toxin-stabilizing peptides (TSP11 and TSP28) was confirmed.

### Example 4: IM LD₅₀ Test of Mixed Formulation of Recombinant Botulinum Neurotoxin Type A and TSP11

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1. This study was conducted after review and approval by the Medytox Animal Experiment Ethics Committee (A-2020-015).

As a test substance, a recombinant botulinum neurotoxin type A identical to the recombinant botulinum neurotoxin type A of Example 2 was used. A toxin-stabilizing peptide (TSP11) was used in a ratio of 46.5 ng/U in the experiment (Table 6). The mixture of the recombinant botulinum neurotoxin type A and the toxin-stabilizing peptide was diluted to concentrations of 300, 450, and 675 U/mL and used in the experiment. The day the test substance was administered was set as day 0, and anesthesia of the test animals and administration of the test substance were performed in the same manner as in Example 1. After administering the test substance to the right gastrocnemius muscle, dead individuals were identified for 14 days. For the dead individuals per each administered dose, IM LD₅₀ values were calculated by PROBIT analysis by using SPSS software 25.0 (SPSS Inc., IL, USA).

**[Table 6]**

| Group | No. of animals | Administered substance and dose | Route of administration | Evaluation indicator |
|---|---|---|---|---|
| 1 | 10 | 30 U/kg eBoNT/A | Right gastrocnemius muscle | Survival |
| 2 | 10 | 90 U/kg eBoNT/A | Right gastrocnemius muscle | Survival |
| 3 | 10 | 135 U/kg eBoNT/A | Right gastrocnemius muscle | Survival |
| 10 | 10 | 60 U/kg eBoNT/A + 46.5 ng/U TSP11 | Right gastrocnemius muscle | Survival |
| 11 | 10 | 90 U/kg eBoNT/A + 46.5 ng/U TSP11 | Right gastrocnemius muscle | Survival |
| 12 | 10 | 135 U/kg eBoNT/A + 46.5 ng/U TSP11 | Right gastrocnemius muscle | Survival |

**[Table 7]**

| Test substance | Median lethal dose by intramuscular administration (U/kg) |
|---|---|
| eBoNT/A | 63.8 |
| eBoNT/A + TSP11 | 81.2 |

As a result of calculating the median lethal dose by observing dead individuals for 14 days after administering the recombinant botulinum neurotoxin type A (eBoNT/A) and the eBoNT/A + TSP11 test substance including the toxin-stabilizing peptide to the right gastrocnemius muscle of the mice as shown in Table 7, the median lethal doses were observed as 63.8 U/kg in the eBoNT/A alone-administered group and as 81.2 U/kg, which is about 1.3-fold increase compared to the eBoNT/A alone-administered group, in the eBoNT/A + TSP11-administered group (Table 7). Based on these results, it was confirmed that safety of the recombinant botulinum neurotoxin was improved by mixing the recombinant botulinum neurotoxin and the toxin-stabilizing peptide (TSP11).

### Example 5: Non-clinical Efficacy Test of Mixed Formulation of Botulinum Neurotoxin Type A and TSP11, TSP17, or TSP19 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1.

Coretox inj. (Medytox) was used as a test substance and a sterilized saline solution (Daehan Pharmaceutical Industry) was used as placebo (placebo-administered group). Toxin-stabilizing peptides (TSP11, TSP17, and TSP19) were synthesized (company name: Anygen Co., Ltd.) and mixed with the botulinum neurotoxin in a ratio of 3×10⁵ per 1 mol of the botulinum neurotoxin and used in the experiment (Table 8). The mixture of the botulinum neurotoxin type and the toxin-stabilizing peptide was diluted to a concentration 120 U/mL and used in the experiment.

The day the test substance was administered was set as day 0, and anesthesia of the test animals and administration of the test substance were performed in the same manner as in Example 1.

For evaluation, the compound muscle action potential (CMAP) test method, which measures the action potential of muscles responding to external electrical stimulation, was used. Specific evaluation methods and statistical analysis were conducted in the same manner as in Example 1.

**[Table 8]**

| Group | No. of animals | Administered substance and dose | Route of administration | Evaluation indicator |
|---|---|---|---|---|
| 1 | 6 | Placebo | Right gastrocnemius muscle | CMAP |
| 2 | 6 | 24 U/kg BoNT/A | Right gastrocnemius muscle | CMAP |
| 3 | 6 | 24 U/kg BoNT/A + 37.9 ng/U TSP17 | Right gastrocnemius muscle | CMAP |
| 4 | 6 | 24 U/kg BoNT/A + 22.2 ng/U TSP19 | Right gastrocnemius muscle | CMAP |
| 5 | 6 | 24 U/kg BoNT/A + 46.5 ng/U TSP11 | Right gastrocnemius muscle | CMAP |

After administering the botulinum neurotoxin type A (BoNT/A) and the BoNT/A + TSP17, BoNT/A + TSP19, and BoNT/A + TSP11 test substances, in which the toxin-stabilizing peptides were mixed, to the right gastrocnemius muscle of the mice at a dose of 24 U/kg, CMAP values were measured at a muscle of the administered site and a contralateral muscle of the non-administered site on day 7 and day 14. As a result, although a significant difference was not observed in the CMAP values of the muscle of the administered site between the BoNT/A alone-administered group and each of the BoNT/A + TSP17-administered group, the BoNT/A + TSP19-administered group, and the BoNT/A + TSP11-administered group, the CMAP values of the contralateral muscle of the BoNT/A + TSP19-administered group and the BoNT/A + TSP11-administered group were observed as significantly high on day 7 and the CMAP values of the contralateral muscle of the BoNT/A + TSP17-administered group and the BoNT/A + TSP11-administered group were as significantly high on day 14, compared to the BoNT/A alone-administered group (FIG. 3). Based on these results, it was confirmed that the effect on reducing diffusion to the contralateral leg was obtained by mixing the botulinum neurotoxin and the toxin-stabilizing peptide (TSP11, TSP17, or TSP19).

### Example 6: Non-clinical Efficacy Test of Mixed Formulation of Botulinum Neurotoxin Type A and TSP11 or TSP28 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1.

Coretox inj. (Medytox) was used as a test substance, and a sterilized saline solution (Daehan Pharmaceutical Industry) was used as placebo (placebo-administered group). Toxin-stabilizing peptides (TSP11 and TSP28) were mixed with the botulinum neurotoxin in a ratio of 3×10⁵ per 1 mol of the botulinum neurotoxin and used in the experiment (Table 9). The mixture of the botulinum neurotoxin and the toxin-stabilizing peptide was diluted to a concentration of 120 U/mL and used in the experiment.

The day the test substance was administered was set as day 0, and anesthesia of the test animals and administration of the test substance were performed in the same manner as in Example 1.

For evaluation, the compound muscle action potential (CMAP) test method, which measures the action potential of muscles responding to external electrical stimulation, was used. Specific evaluation methods and statistical analysis were conducted in the same manner as in Example 1.

**[Table 9]**

| Group | No. of animals | Administered substance and dose | Route of administration | Evaluation indicator |
|---|---|---|---|---|
| 1 | 6 | Placebo | Right gastrocnemius muscle | CMAP |
| 2 | 6 | 24 U/kg BoNT/A | Right gastrocnemius muscle | CMAP |
| 3 | 6 | 24 U/kg BoNT/A + 46.5 ng/U TSP11 | Right gastrocnemius muscle | CMAP |
| 4 | 6 | 24 U/kg BoNT/A + 38.3 ng/U TSP28 | Right gastrocnemius muscle | CMAP |

After administering the botulinum neurotoxin type A (BoNT/A) and the BoNT/A + TSP11 and BoNT/A + TSP24 test substances, in which the toxin-stabilizing peptides were mixed, to the right gastrocnemius muscle of the mice at a dose of 24 U/kg, CMAP values were measured at a muscle of the administered site and a contralateral muscle of the non-administered site on day 7 and day 14. As a result, although a significant difference was not observed in the CMAP values of the muscle of the administered site between the BoNT/A alone-administered group and each of the BoNT/A + TSP11-administered group and the BoNT/A + TSP28-administered group, the CMAP values of the contralateral muscle of the BoNT/A + TSP11-administered group and the BoNT/A + TSP28-administered group were observed as significantly high on both day 7 and day 14, compared to the BoNT/A alone-administered group (FIG. 4). Based on these results, it was confirmed that the effect on reducing diffusion to the contralateral leg was obtained by mixing the botulinum neurotoxin and the toxin-stabilizing peptides (TSP11 and TSP28).

### Example 7: Non-clinical Efficacy Test of Mixed Formulation of Botulinum Neurotoxin Type A 19S and TSP11 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1.

As a test substance, 19S non-albumin formulation (Medytox; formulation including 19S toxin instead of 7S toxin of Coretox), and a sterilized saline solution (Daehan Pharmaceutical Industry) was used as placebo (placebo-administered group). A toxin-stabilizing peptide (TSP11) was mixed with the botulinum neurotoxin in a ratio of 3×10⁵ per 1 mole of the botulinum neurotoxin and used in the experiment (Table 10). The mixture of the botulinum neurotoxin and the toxin-stabilizing peptide was diluted to a concentration of 120 U/mL and used in the experiment.

The day the test substance was administered was set as day 0, and anesthesia of the test animals and administration of the test substance were performed in the same manner as in Example 1. For evaluation, the compound muscle action potential (CMAP) test method, which measures the action potential of muscles responding to external electrical stimulation, was used. Specific evaluation methods and statistical analysis were conducted in the same manner as in Example 1.

**[Table 10]**

| Group | No. of animals | Administered substance and dose | Route of administration | Evaluation indicator |
|---|---|---|---|---|
| 1 | 6 | Placebo | Right gastrocnemius muscle | CMAP |
| 2 | 6 | 24 U/kg BoNT/A 19S | Right gastrocnemius muscle | CMAP |
| 3 | 6 | 24 U/kg BoNT/A 19S + 46.5 ng/U TSP11 | Right gastrocnemius muscle | CMAP |

After administering the botulinum neurotoxin type A 19S (BoNT/A) and the BoNT/A 19S + TSP11 test substance, in which the toxin-stabilizing peptide was mixed, to the right gastrocnemius muscle of the mice at a dose of 24 U/kg, CMAP values were measured at a muscle of the administered site and a contralateral muscle of the non-administered site on day 7 and day 14. As a result, although a significant difference was not observed in the CMAP values of the muscle of the administered site between the BoNT/A 19S alone-administered group and the BoNT/A 19S + TSP11-administered group, the CMAP values of the contralateral muscle was observed as significantly high in the BoNT/A 19S + TSP11-administered group on day 14, compared to the BoNT/A 19S alone-administered group (FIG. 5). Based on these results, it was confirmed that the effect on reducing diffusion to the contralateral leg was obtained by mixing the botulinum neurotoxin 19S and the toxin-stabilizing peptide (TSP11).

### Example 8: Non-clinical Efficacy Test of Mixed Formulation of Botulinum Neurotoxin Type A and TSP1 or TSP2 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1.

Coretox inj. (Medytox) was used as a test substance, and a sterilized saline solution (Daehan Pharmaceutical Industry) was used as placebo (placebo-administered group). Toxin-stabilizing peptides (TSP1 and TSP2) was mixed with the botulinum neurotoxin in a ratio of 1×10⁶ per 1 mol of the botulinum neurotoxin and used in the experiment (Table 11). The mixture of the botulinum neurotoxin and the toxin-stabilizing peptide was diluted to a concentration of 60 U/mL and used in the experiment.

The day the test substance was administered was set as day 0, and anesthesia of the test animals and administration of the test substance were performed in the same manner as in Example 1.

For evaluation, the compound muscle action potential (CMAP) test method, which measures the action potential of muscles responding to external electrical stimulation, was used. Specific evaluation methods and statistical analysis were conducted in the same manner as in Example 1.

**[Table 11]**

| Group | No. of animals | Administered substance and dose | Route of administration | Evaluation indicator |
|---|---|---|---|---|
| 1 | 6 | Placebo | Right gastrocnemius muscle | CMAP |
| 2 | 6 | 12 U/kg BoNT/A | Right gastrocnemius muscle | CMAP |
| 3 | 6 | 12 U/kg BoNT/A + 169.7 ng/U TSP1 | Right gastrocnemius muscle | CMAP |
| 4 | 6 | 12 U/kg BoNT/A + 137 ng/U TSP2 | Right gastrocnemius muscle | CMAP |

After administering the botulinum neurotoxin type A (BoNT/A) and the BoNT/A + TSP1 and BoNT/A + TSP2 test substances, in which the toxin-stabilizing peptides were mixed, to the right gastrocnemius muscle of the mice at a dose of 12 U/kg, CMAP values were measured at a muscle of the administered site and a contralateral muscle of the non-administered site on day 7 and day 14. As a result, although a significant difference in the CMAP values at the muscle of the administered site was not observed between the BoNT/A alone-administered group and each of the BoNT/A + TSP1-administered group and the BoNT/A + TSP2-administered group, a significantly higher CMAP value of the contralateral muscle of the BoNT/A + TSP2-administered group than that of the BoNT/A alone-administered group was observed (FIG. 6). On the contrary, a significant difference in the CMAP values was not observed in the contralateral muscle of the BoNT/A + TSP1-administered group, compared wo the BoNT/A alone-administered group. Based on these results, it was confirmed that the effect on reducing diffusion to the contralateral leg was obtained by mixing the botulinum neurotoxin and TSP2.

### Example 9: Non-clinical Efficacy Test of Mixed Formulation of Botulinum Neurotoxin Type A and TSP11 or TSP23 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1.

Coretox inj. (Medytox) was used as a test substance, and a sterilized saline solution (Daehan Pharmaceutical Industry) was used as placebo (placebo-administered group). Toxin-stabilizing peptides (TSP11 and TSP23) were mixed with the botulinum neurotoxin in a ratio of 3×10⁵ per 1 mol of the botulinum neurotoxin and used in the experiment (Table 12). The mixture of the botulinum neurotoxin and the toxin-stabilizing peptide was diluted to a concentration of 120 U/mL and used in the experiment.

The day the test substance was administered was set as day 0, and anesthesia of the test animals and administration of the test substance were performed in the same manner as in Example 1.

For evaluation, the compound muscle action potential (CMAP) test method, which measures the action potential of muscles responding to external electrical stimulation, was used. Specific evaluation methods and statistical analysis were conducted in the same manner as in Example 1.

**[Table 12]**

| Group | No. of animals | Administered substance and dose | Route of administration | Evaluation indicator |
|---|---|---|---|---|
| 1 | 6 | Placebo | Right gastrocnemius muscle | CMAP |
| 2 | 6 | 24 U/kg BoNT/A | Right gastrocnemius muscle | CMAP |
| 3 | 6 | 24 U/kg BoNT/A + 46.5 ng/U TSP11 | Right gastrocnemius muscle | CMAP |
| 4 | 6 | 24 U/kg BoNT/A + 44.8 ng/U TSP23 | Right gastrocnemius muscle | CMAP |

After administering the botulinum neurotoxin type A (BoNT/A) and the BoNT/A + TSP11 and BoNT/A + TSP23 test substances, in which the toxin-stabilizing peptides were mixed, to the right gastrocnemius muscle of the mice at a dose of 24 U/kg, CMAP values were measured at a muscle of the administered site and a contralateral muscle of the non-administered site on day 7 and day 14. As a result, although a significant difference was not observed in the CMAP values of the muscle of the administered site between the BoNT/A alone-administered group and the BoNT/A + TSP11-administered group, a significant difference was confirmed between the BoNT/A alone-administered group and the BoNT/A + TSP23-administered group. The CMAP values of the contralateral muscle of the BoNT/A + TSP11-administered group and the BoNT/A + TSP23-administered group were observed as significantly high compared to that of the BoNT/A alone-administered group (FIG. 7).

### Example 10: Non-clinical Efficacy Test of Mixed Formulation of Botulinum Neurotoxin Type A and TSP11 or TSP35 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1.

Coretox inj. (Medytox) was used as a test substance, and a sterilized saline solution (Daehan Pharmaceutical Industry) was used as placebo (placebo-administered group). Toxin-stabilizing peptides (TSP11 and TSP35) were mixed with the botulinum neurotoxin in a ratio of 3×10⁵ per 1 mol of the botulinum neurotoxin and used in the experiment (Table 13). The mixture of the botulinum neurotoxin and the toxin-stabilizing peptide was diluted to a concentration of 120 U/mL and used in the experiment.

The day the test substance was administered was set as day 0, and anesthesia of the test animals and administration of the test substance were performed in the same manner as in Example 1.

For evaluation, the compound muscle action potential (CMAP) test method, which measures the action potential of muscles responding to external electrical stimulation, was used. Specific evaluation methods and statistical analysis were conducted in the same manner as in Example 1.

**[Table 13]**

| Group | No. of animals | Administered substance and dose | Route of administration | Evaluation indicator |
|---|---|---|---|---|
| 1 | 6 | Placebo | Right gastrocnemius muscle | CMAP |
| 2 | 6 | 24 U/kg BoNT/A | Right gastrocnemius muscle | CMAP |
| 3 | 6 | 24 U/kg BoNT/A + 46.5 ng/U TSP11 | Right gastrocnemius muscle | CMAP |
| 4 | 6 | 24 U/kg BoNT/A + 38.0 ng/U TSP35 | Right gastrocnemius muscle | CMAP |

After administering the botulinum neurotoxin type A (BoNT/A) and the BoNT/A + TSP11 and BoNT/A + TSP35 test substances, in which the toxin-stabilizing peptides were mixed, to the right gastrocnemius muscle of the mice at a dose of 24 U/kg, CMAP values were measured at a muscle of the administered site and a contralateral muscle of the non-administered site on day 7 and day 14. As a result, although significant differences in the CMAP values at the muscle of the administered site were observed between the BoNT/A alone-administered group and the BoNT/A + TSP11-administered group and between the BoNT/A alone-administered group and the BoNT/A + TSP35-administered group on day 7, a significant difference was observed only between the BoNT/A alone-administered group and the BoNT/A + TSP35-administered group on day 14. On the contrary, significant differences in the CMAP values of the contralateral muscle of the non-administered site were observed on both day 7 and day 14 between the BoNT/A alone-administered group and each of the BoNT/A + TSP11-administered group and the BoNT/A + TSP35-administered group (FIG. 8).

### Example 11: Non-clinical Efficacy Test of Mixed Formulation of Botulinum Neurotoxin Type A and TSP11 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1.

Coretox inj. (Medytox) was used as a test substance, and a sterilized saline solution (Daehan Pharmaceutical Industry) was used as placebo (placebo-administered group). A toxin-stabilizing peptide (TSP11) was mixed with the botulinum neurotoxin in a ratio of 3×10⁵ per 1 mol of the botulinum neurotoxin and used in the experiment (Table 14). The mixture of the botulinum neurotoxin and the toxin-stabilizing peptide was diluted to concentrations of 30 and 60 U/mL and used in the experiment.

The day the test substance was administered was set as day 0, and anesthesia of the test animals and administration of the test substance were performed in the same manner as in Example 1.

For evaluation, the digit abduction scoring (DAS) assay, which visually evaluates the degree of muscle paralysis in mice, was performed. The DAS values shown in Table 15 indicate the degree of muscle paralysis according to the shape of toes of the administered site in the mice. A DAS recovery period (duration of action of botulinum neurotoxin) refers to a period of time during which the DAS return to 0 after administration of a test substance. Weights were measured once a week.

**[Table 14]**

| Group | No. of animals | Administered substance and dose | Route of administration | Evaluation indicator |
|---|---|---|---|---|
| 1 | 8 | Placebo | Right gastrocnemius muscle | DAS |
| 2 | 8 | 3 U/kg BoNT/A | Right gastrocnemius muscle | DAS |
| 3 | 8 | 12 U/kg BoNT/A + 46.5 ng/U TSP11 | Right gastrocnemius muscle | DAS |

**[Table 15]**

| Score | Criteria |
|---|---|
| 0 | Normal, no difference from the shape of the foot on the non-injected side |
| 1 | Space between the toes is narrowed, or two toes are close together and the rest are fully extended |
| 2 | All toes are significantly narrowed, or three toes are stuck together |
| 3 | Feet are curved and four toes are stuck together |
| 4 | Feet are curved and all toes are stuck together |

**[Table 16]**

| | Average weight change based on weight on week 0 at each measurement point (%) | | | | | | | |
|---|---|---|---|---|---|---|---|---|
| | Week 0 | Week 1 | Week 2 | Week 3 | Week 4 | Week 5 | Week 6 | Week 7 |
| Placebo | 100 | 105.4 | 109.5 | 116.9 | 122 | 124.8 | 131.6 | 132.1 |
| 3 U/kg BoNT/A | 100 | 99.8 | 102.7 | 111.1 | 113.5 | 124.6 | 125.7 | 126.3 |
| 12 U/kg BoNT/A + TSP11 | 100 | 99.9 | 103.4 | 114.3 | 114.7 | 120.1 | 126.3 | 126.7 |

After administering the 6 U/kg botulinum neurotoxin type A (BoNT/A) and the BoNT/A + TSP11 test substance, in which the botulinum neurotoxin type A was mixed with 12 U/kg toxin-stabilizing peptide, to the right gastrocnemius muscle of the mice, DAS and weights were measured for 7 weeks. As a result of measuring variation in weights at each week based on the weight at the time of administration of the test substance (week 0), no significant difference was observed between the 6 U/kg BoNT/A-administered group and the 12 U/kg BoNT/A + TSP11-administered group (Table 16). In addition, DAS recovery was observed at day 23.8 on average in the 6 U/kg BoNT/A-administered group, and DAS recovery was observed at day 35.6 on average in the 12 U/kg BoNT/A + TSP11-administered group indicating an approximately 1.5-fold increase in the duration of action compared to the BoNT/A alone-administered group (FIG. 9). Based on these results, it was confirmed that the duration of action of the botulinum neurotoxin may be increased without changing safety by increasing the dose of the botulinum neurotoxin in a mixed formulation of the botulinum neurotoxin and the toxin-stabilizing peptide (TSP11).

### Example 12: Non-clinical Efficacy Test of Mixed Formulation of Botulinum Neurotoxin Type A and TSP47 or TSP48 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1. This study was conducted after review and approval by the Medytox Animal Experiment Ethics Committee (A-2022-007).

Coretox inj. (Medytox) was used as a test substance, and a sterilized saline solution (Daehan Pharmaceutical Industry) was used as placebo (placebo-administered group. Toxin-stabilizing peptides (TSP47 and TSP48) were mixed with the botulinum neurotoxin in a ratio of 3×10⁵ per 1 mol of the botulinum neurotoxin and used in the experiment (Table 17). The mixture of the botulinum neurotoxin and the toxin-stabilizing peptide was diluted to a concentration of 120 U/mL and used in the experiment.

The day the test substance was administered was set as day 0, and anesthesia of the test animals and administration of the test substance were performed in the same manner as in Example 1.

For evaluation, the compound muscle action potential (CMAP) test method, which measures the action potential of muscles responding to external electrical stimulation, was used. Specific evaluation methods and statistical analysis were conducted in the same manner as in Example 1.

**[Table 17]**

| Group | No. of animals | Administered substance and dose | Route of administration | Evaluation indicator |
|---|---|---|---|---|
| 1 | 6 | Placebo | Right gastrocnemius muscle | CMAP |
| 2 | 6 | 24 U/kg BoNT/A | rRight gastrocnemius muscle | CMAP |
| 3 | 6 | 24 U/kg BoNT/A + 46.49 ng/U TSP47 | Right gastrocnemius muscle | CMAP |
| 4 | 6 | 24 U/kg BoNT/A + 38.33 ng/U TSP48 | Right gastrocnemius muscle | CMAP |

After administering the botulinum neurotoxin type A (BoNT/A) and the BoNT/A + TSP47 test substance and the BoNT/A + TSP48 test substance, in which the toxin-stabilizing peptides were mixed, to the right gastrocnemius muscle of the mice at a dose of 24 U/kg, CMAP values were measured at a muscle of the administered site and a contralateral muscle of the non-administered site on day 7 and day 14. As a result, although a significant increase in the CMAP value of the muscle of the administered site was observed in the BoNT/A + TSP47-administered group on day 7 after administration compared to the BoNT/A alone-administered group, a significant difference was not observed on day 14 compared to the BoNT/A alone-administered group. On the contrary, the CMAP values of the contralateral muscle were observed as significantly high on day 7 and day 14 both in the BoNT/A + TSP47-administered group and the BoNT/A + TSP48-administered group, compared to the BoNT/A alone-administered group. Based on these results, it was confirmed that the effect on reducing diffusion to the contralateral leg was obtained by mixing the botulinum neurotoxin and TSP47 or TSP 48 (FIG. 10).

### Example 13: Non-clinical Efficacy Test of Mixed Formulation of Botulinum Neurotoxin Type A and TSP56 or TSP61 in Mice

In the same manner as in Example 1, 5-week-old female CD1 (ICR) mice were purchased from Orient Bio Co., Ltd., and after acclimatization and quarantine for 1 week, 6-week-old mice were used in the experiment. The feed, drinking water, and breeding conditions were the same as those given in Example 1. This study was conducted after review and approval by the Medytox Animal Experiment Ethics Committee (A-2022-007).

Coretox inj. (Medytox) was used as a test substance, and a sterilized saline solution (Daehan Pharmaceutical Industry) was used as placebo (placebo-administered group. Toxin-stabilizing peptides (TSP56 and TSP61) were mixed with the botulinum neurotoxin in a ratio of 3×10⁵ per 1 mol of the botulinum neurotoxin and used in the experiment (Table 18). The mixture of the botulinum neurotoxin and the toxin-stabilizing peptide was diluted to a concentration of 120 U/mL and used in the experiment.

The day the test substance was administered was set as day 0, and anesthesia of the test animals and administration of the test substance were performed in the same manner as in Example 1.

For evaluation, the compound muscle action potential (CMAP) test method, which measures the action potential of muscles responding to external electrical stimulation, was used. Specific evaluation methods and statistical analysis were conducted in the same manner as in Example 1.

**[Table 18]**

| Group | No. of animals | Administered substance and dose | Route of administration | Evaluation indicator |
|---|---|---|---|---|
| 1 | 6 | 24 U/kg BoNT/A | Right gastrocnemius muscle | CMAP |
| 2 | 6 | 24 U/kg BoNT/A + 45.85 ng/U TSP56 | Right gastrocnemius muscle | CMAP |
| 3 | 6 | 24 U/kg BoNT/A + 46.18 ng/U TSP61 | Right gastrocnemius muscle | CMAP |

After administering the botulinum neurotoxin type A (BoNT/A) and each of the BoNT/A + TSP56 test substance and the BoNT/A + TSP61 test substance, in which the toxin-stabilizing peptides were mixed, to the right gastrocnemius muscle of the mice at a dose of 24 U/kg, CMAP values were measured at a muscle of the administered site and a contralateral muscle of the non-administered site on day 7 and day 14. As a result, significant increases in the CMAP values of the muscle of the administered site were observed on day 7 and day 14 after administration in the BoNT/A + TSP56-administered group compared to the BoNT/A alone-administered group. However, the CMAP values of the contralateral muscle in the BoNT/A + TSP56-administered group were observed as significantly high on day 7 and day 14 compared to the BoNT/A alone-administered group. In the case where the botulinum neurotoxin was mixed with TSP56, the CMAP value was slightly increased in the muscle of the administered site compared to the botulinum neurotoxin alone-administered group, but the CMAP value was more increased in the contralateral leg, so that the diffusion-reducing effect obtained by mixing with TSP 56 was confirmed. Although the CMAP value of the muscle of the administered site of the BoNT/A + TSP61-administered group was not significantly different from that of the BoNT/A alone-administered group, the CMAP value of the contralateral muscle thereof was observed as significantly high compared to that of the BoNT/A alone-administered group, so that the diffusion-reducing effect obtained by mixing with TSP 61 was confirmed.

### Sequence List Text

SEQ ID NOS: 1 to 10 are exemplary sequences of moieties that interact with a cell membrane of a nerve cell and interacting with the botulinum neurotoxin. SEQ ID NOS: 11 to 13 are exemplary sequences of moieties that interact with a cell membrane of a nerve cell. SEQ ID NOS: 14 to 21 are exemplary sequences of polypeptides included in the composition for stabilizing a botulinum neurotoxin. SEQ ID NO: 22 is a substance used as a control material of the polypeptide included in the toxin stabilizing composition according to an embodiment. SEQ ID NO: 23 is a sequence of the recombinant botulinum neurotoxin type A light chain. SEQ ID NO: 24 is a sequence of the recombinant botulinum neurotoxin type A in which the light chain of the botulinum neurotoxin consists of a combination of BoNT/A1 light chain and BoNT/A4 light chain.

## Claims

1. A composition for stabilizing a botulinum neurotoxin comprising a polypeptide inhibiting diffusion of the botulinum neurotoxin *in vivo* and represented by Formula 1 below:
[Formula 1] (C)ₙ-V
wherein in the formula, C represents a moiety interacting with a cell membrane of a nerve cell, V represents a moiety interacting with a cell membrane of a nerve cell and interacting with the botulinum neurotoxin, and n is an integer of 0 or 1.

2. The composition of claim 1, wherein V is a VAMP (vesicle associated membrane protein), a BDNF (brain-derived neurotrophic factor), a fragment thereof, or a variant thereof.

3. The composition of claim 1 or 2, wherein V is VpN of VAMP, a fragment thereof, or a variant thereof.

4. The composition of claim 2 or 3, wherein VAMP is VAMP1, VAMP2, VAMP3, a fragment thereof, or a variant thereof.

5. The composition of any one of claims 1 to 4, wherein V comprises the amino acid sequence of SEQ ID NO: 1.

6. The composition of claim 5, wherein V is selected from: amino acid sequences of SEQ ID NOS: 1, 2, 3, 4, 5, 6, 7, and 8; and amino acid sequences of SEQ ID NOS: 1, 2, 3, 4, 5, 6, 7, and 8 of which one amino acid is substituted.

7. The composition of any one of claims 1 to 6, wherein C is a cell penetrating peptide or a cationic peptide.

8. The composition of claim 7, wherein C is an arginine-rich peptide.

9. The composition of claim 7 or 8, wherein C further comprises an aromatic amino acid.

10. The composition of claim 9, wherein the aromatic amino acid is tryptophan.

11. The composition of any one of claims 7 to 10, wherein C comprises 6 to 9 arginines and 2 to 3 tryptophans.

12. The composition of any one of claims 7 to 11, wherein C has a three-dimensional structure with arginine and tryptophan biased toward one side.

13. The composition of any one of claims 7 to 12, wherein C consists of the amino acid sequence of SEQ ID NO: 11, SEQ ID NO: 12, or SEQ ID NO: 13.

14. The composition of any one of claims 1 to 13, wherein the polypeptide consists of the amino acid sequence of SEQ ID NO: 8, SEQ ID NO: 14, SEQ ID NO: 15, SEQ ID NO: 16, SEQ ID NO: 17, SEQ ID NO: 18, SEQ ID NO: 19, SEQ ID NO: 20, or SEQ ID NO: 21.

15. A botulinum neurotoxin formulation comprising a botulinum neurotoxin and the composition for stabilizing a botulinum neurotoxin of any one of claims 1 to 14.

16. The botulinum neurotoxin formulation of claim 15, wherein the botulinum neurotoxin is type A, B, C, D, E, F, or G.

17. The botulinum neurotoxin formulation of claim 15 or 16, wherein the botulinum neurotoxin is a naturally occurring or a recombinant toxin.

18. The botulinum neurotoxin formulation of any one of claims 15 to 17, wherein the botulinum neurotoxin is in the form of 7S or 19S.

19. The botulinum neurotoxin formulation of any one of claims 16 to 19, comprising an increased amount of the botulinum neurotoxin compared to a case not including a composition for stabilizing a botulinum neurotoxin.

20. A polypeptide consisting of the amino acid sequence of SEQ ID NO: 15, SEQ ID NO: 16, SEQ ID NO: 17 SEQ ID NO: 18, SEQ ID NO: 19, or SEQ ID NO: 20.
